# EUROPEAN PATENT APPLICATION

(11) **EP 3 412 261 A1**
(43) Date of publication of application: **12.12.2018**
(21) Application number: 16889495.4
(22) Date of filing: 28.04.2016
(51) Int. Cl.: A61F 9/008, H01S 3/00, G02B 7/02

(54) **VISION CORRECTION SURGERY LASER ADAPTOR WHICH CREATES LASER BEAM NON-IRRADIATED REGION**

(30) Priority: 03.02.2016 KR 20160013299
(71) Applicant: Jung, Young Taek, Jeonju-si, Jeollabuk-do 54997 (KR)
(72) Inventor: Jung, Young Taek, Jeonju-si, Jeollabuk-do 54997 (KR)
(74) Representative: Dilg, Haeusler, Schindelmann Patentanwaltsgesellschaft mbH
(86) International application number: PCT/KR2016/004446
(87) International publication number: WO 2017/135517

(57) **Abstract**

The present invention relates to an adaptor of a laser for use in vision correction surgery that forms a laser beam non-irradiated region, of which an upper portion is coaxially aligned with an object lens of the laser, and a lower portion is coaxially aligned with a cornea of a patient, in order to set a position of the cornea of the patient with respect to the object lens of the laser. A pair of spaced shields for blocking the laser beam is provided on a non-refraction lens or refraction lens which is installed in the adaptor, the laser beam of the laser going through the non-refraction lens or refraction lens

## Description

### [Technical Field]

The present invention relates to an adaptor of a laser for use in vision correction surgery that connects an object lens of the laser and a patient's cornea so that a laser beam irradiated from the laser can be accurately irradiated on the patient's cornea.

### [Background Art]

The following background art is provided only for easy understanding of the present invention, and therefore it should be noted that the background art is not regarded as the related art.

A cornea is located at the outermost surface of an eyeball, through which light is first transmitted. The cornea is a front transparent tissue of the eye which refracts the light to form an image on a retina. The retina is a rear tissue of the eye which senses the light from the image formed thereon and transmits a signal based on the image to the brain.

Meanwhile, a method of correcting imperfect vision of the eyeball includes vision correction surgery for changing a thickness of the cornea of the eyeball to correct the imperfect vision of the eyeball. Typical examples of the vision correction surgery are laser in-situ keratomileusis (LASIK), laser assisted sub-epithelial keratomileusis (LASEK), and small incision lenticule extraction (SMILE).

The small incision lenticule extraction (SMILE) surgery is to correct abnormal refraction by incising a portion of a corneal stroma inside the surface of the cornea using a laser. In the SMILE surgery, a portion of the corneal stroma that needs to be corrected is accurately cut in the shape of a lens in accordance with a desired amount of correction in the cornea using a precise VisuMax femtosecond laser, and then the cut portion of the corneal stroma is removed through an incision portion formed in the cornea without forming a corneal flap, unlike the LASIK surgery and the LASEK surgery. Consequently, the SMILE surgery has advantages in that damage to surrounding tissues is reduced, high precision is achieved, and the risk of side effects, such as myopic regression, congestion, and xerophthalmia, is remarkably reduced.

The above SMILE surgery is regarded as the same kind of surgery even though various kinds of lasers are used and various names are used depending on the manufacturer.

Hereinafter, the SMILE surgery will be described in brief. If laser beam irradiated from the laser is transmitted through the surface of the cornea, and is applied to a corneal stroma, a lenticule, which is a piece of the corneal stroma, is cut from the corneal stroma in the shape of a lens in accordance with a desired amount of correction. And then, an incision portion is formed in the cornea using the laser beam, and the cut lenticule is removed out of the cornea through the incision portion.

The incision portion has an incision entrance formed on the surface of the cornea and an incision tunnel extending from the incision entrance toward the lenticule. The lenticule is removed out of the cornea through the incision tunnel and the incision entrance.

Meanwhile, when the lenticule is cut from the corneal stroma using the laser beam, tissue bridges, which are uncut regions, are formed between spots of the laser beam, since the spots of the laser beam are circular.

Even in case where the lenticule is three-dimensionally cut from the corneal stroma using the laser beam, a flap surface which is a separated upper surface of the lenticule, and a lenticular surface which is a separated lower surface of the lenticule are not completely separated from the corneal stroma due to the tissue bridges. For this reason, a separation tool, such a long needle, is inserted through the incision entrance and the incision tunnel such that the separation tool moves along the flap surface and the lenticular surface to completely separate the lenticule from the corneal stroma. And then, a picking tool, such as a pair of tweezers, is inserted through the incision entrance and the incision tunnel to remove the lenticule, to the outside through the incision entrance while holding the lenticule.

However, the incision entrance and the incision tunnel are incised in lengths of about 2 mm to 4 mm. When the separation tool is inserted into the center of the cornea through the incision entrance and the incision tunnel, and is rotated along the lenticular surface and the flap surface in alternating directions to separate the lenticule which is connected to the corneal stroma via the tissue bridges, from the corneal stroma, both sides of the incision portion is applied by an external force generated at rotation of the separation tool.

In case where the incision entrance and the incision tunnel are formed in lengths of about 2 mm, both inner sides of the incision entrance and the incision tunnel may be easily torn by the external force generated as the result of motion of the separation tool. Therefore, after the incision entrance and the incision tunnel are formed in lengths of about 3.5 mm or more, the surgery is generally executed.

In addition, even though the incision portion is formed in lengths of 2 mm or less, and the lenticule is then separated from the corneal stroma while the separation tool is being carefully moved, the higher the abnormal refraction, the thicker the lenticule that must be cut from the corneal stroma. Both sides of the incision entrance and the incision tunnel easily are torn, in case of withdrawing the thick lenticule from the corneal stroma.

Therefore, since it is preferable that the risk of corneal infection is reduced, and the length of the incision portion formed on the cornea is minimized so that medical treatment time is reduced and tissues are stabilized, there is highly necessary for a method capable of preventing both sides of the incision entrance and the incision tunnel from being easily torn while forming the incision entrance and the incision tunnel in lengths of about 2 mm or less.

### [Disclosure]

### [Technical Problem]

Accordingly, the present invention has been made in view of the above problems occurring in the related art, and it is an object of the present invention to provide an adaptor of a laser for use in vision correction surgery that forms a laser beam non-irradiated region, wherein as well as minimizing a length of an incision portion formed on a cornea to remove a cut lenticule from an inside of the cornea by laser for vision correction surgery, both sides of the incision portion are prevented from being torn, thereby reducing a risk of corneal infection, and a degree of completion in the surgery is further improved by quick regeneration and stabilization of tissues, in addition to a shorten effect of medical treatment.

### [Technical Solution]

To accomplish the above object, according to one aspect of the present invention, there is provided an adaptor of a laser for use in vision correction surgery that forms a laser beam non-irradiated region, of which an upper portion is coaxially aligned with an object lens of the laser, and a lower portion is coaxially aligned with a cornea of a patient, in order to set a position of the cornea of the patient with respect to the object lens of the laser, wherein a pair of spaced shields for blocking downward penetration of the laser beam is provided on a non-refraction lens or refraction lens which is installed in the adaptor, the laser beam of the laser going through the non-refraction lens or refraction lens.

Preferably, a focal point of the laser beam is three-dimensionally displaced to continuously draw dense helices on an inside of the cornea, so that a flap surface and a lenticular surface which have a continuous spiral incision line are formed in a corneal stroma. An outer circumference of the lenticular surface intersects at any point inside an outer circumference of the flap surface, and the laser beam non-irradiated region is formed by the shield when a three-dimensional lenticule is incised from the corneal stroma. The laser beam non-irradiated region is formed on any one of the incision line of the flap surface which is positioned between outer circumferential incision lines, among outer circumferential incision lines of the flap surface.

Preferably, an incision entrance having a desired length in a circumferential direction is formed on the surface of the cornea which is opposite to the incision line, by the laser beam irradiated from the laser, a front end of an incision tunnel formed toward the corneal stroma is connected to the incision line of the flap surface, and the front end of the incision tunnel is positioned between the laser beam non-irradiated regions positioned on the incision line of the flap surface.

Preferably, the incision entrance is formed in the surface of the cornea which is positioned on any one of a vertical line or a line of an acute or obtuse angle relative to the outer circumferential incision line of the flap surface.

Preferably, the shield is formed on a bottom surface of the non-refraction lens or refraction lens.

Preferably, the shield includes any one of a coating layer, a printed layer, a etched portion, and an adhesive substance.

Preferably, the shield includes any one of a pair of circular shields, a pair of oval shields, a pair of polygonal shields, a pair of straight shields, and a pair of curved shields.

Preferably, an interval between the spaced shields has a length of 0.5 mm to 4 mm.

Preferably, the shield includes a pair of straight shields which is horizontal to each other or has an inclined angle while being symmetrical to each other.

Preferably, the shield is formed in such a way that an inner portion of an end facing a center protrudes in an inward direction.

### [Advantageous Effects]

According to the present invention, when the vision correction surgery is executed by three-dimensionally incising a portion of the corneal stroma by the laser, the shields are formed on the adaptor connecting the laser and the cornea to correctly guide the laser beam irradiated from the laser to the cornea of the patient. Therefore, when the incision portion is formed by irradiating the laser beam onto the cornea, the laser beam non-irradiated regions corresponding to the shape of the shields are formed on both sides of the incision portion. When the separation tool is inserted and rotated in the incision portion to remove the lenticule, it is possible to prevent both sides of the incision portion from being easily torn even though the external force is applied to both sides of the incision portion. Also, since the laser beam non-irradiated region is provided, the length of the incision portion is minimized, thereby reducing the risk of corneal infection, reducing the medical treatment time, and rapidly regenerating and stabilizing tissues.

### [Description of Drawings]

Fig. 1a is a side view illustrating a configuration of an adaptor for connecting a laser and a cornea according to one embodiment of the present invention;
Fig. 1b is a bottom view of Fig. la;
Fig. 2a is a side view illustrating a configuration of an adaptor for connecting a laser and a cornea according to another embodiment of the present invention;
Fig. 2b is a bottom view of Fig. 2a;
Fig. 3 is a plane view of an eye cornea having a laser beam non-irradiated region according to one embodiment of the present invention;
Fig. 4 is a side view illustrating a state of an eye cornea, in which a lenticule is incised, according to one embodiment of the present invention; and
Fig. 5 is a plane view illustrating a process of separating a flap surface and a lenticule surface from a corneal stroma by a separation tool inserted in the corneal stroma through an incision portion of a cornea according to one embodiment of the present invention.

### [Mode for Invention]

Reference will now be made in detail to preferred embodiments, examples of which are illustrated in the accompanying drawings.

It should be noted herein that these embodiments are given only for illustrative purposes such that the present invention can be easily embodied by those skilled in the art to which the present invention pertains, and the technical thought and scope of the present invention are not limited thereto.

In the drawings, sizes and shapes of elements may be exaggerated for convenience and clarity of description. In addition, terms specially defined in consideration of the construction and operation of the present invention may be varied depending upon intentions of users or operators or usual practices. The definition of such terms must be made based on the disclosure of the present invention.

First of all, an adaptor of a laser for use in vision correction surgery that forms a laser beam non-irradiated region in a cornea will be described.

The adaptor is not necessary to have any one specific shape, but the adaptor may be formed in such a way that an upper portion is fixed to an object lens (not illustrated), while a lower portion is fixed to a cornea 400 of a patient.

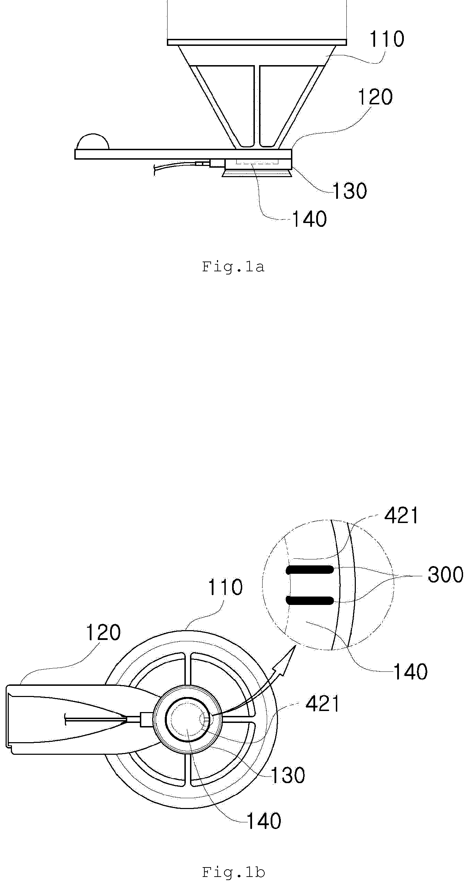

According to one embodiment of the adaptor shown in Figs. 1a and 1b, an upper portion of the adaptor is coaxially fitted and engaged to an object lens, and is provided at a lower portion with a fixture 110 having a lens at a center, through which laser beam passes, a cramp 120 of a tweezers type which is coaxially arranged with the fixture and is engaged to the texture at an upper portion, and an annular ocular ring 130 which is coaxially engaged to a lower portion of the cramp 120 and is put on the cornea 400 of the patient.

One side of the ocular ring 130 is provided with a hose connected to a vacuum generator (not illustrated), such as a syringe, so that the ocular ring is sucked and fixed on the cornea 400 by vacuum generated around an inner peripheral surface of the ocular ring 130.

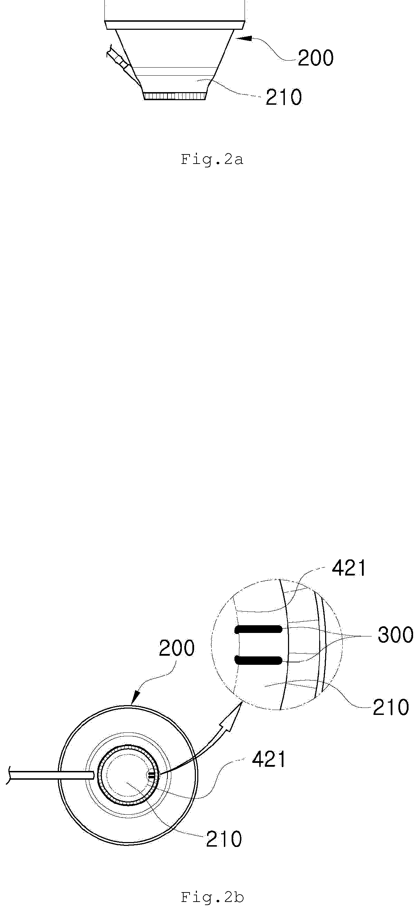

According to another embodiment of the adaptor in Figs. 2 and 2b, an upper portion of the adaptor has one body 200 which is coaxially engaged to the cornea 400 of the patient, and the body 200 has a lens at a center through which the laser beam passes.

An upper end of the body 200 is sucked and fixed by the vacuum generator (not illustrated) provided to the object lens in a state being fitted into the object lens, and one side of an lower end of the body 200 is provided with a hose connected to the vacuum generator, such as a syringe, so that the lower end of the body 200 is sucked and fixed on the cornea 400 by vacuum generated around an inner peripheral surface of the lower end of the body 200.

The laser beam passes through a top surface of the lens which is installed on the fixture 110 and the body 200, and a bottom surface of the lens adheres to the surface of the cornea 400. Such a lens may be a non-refraction lens of flat top and bottom surfaces, or may be a refraction lens of which a top surface is flat, while a bottom surface is concave.

The laser with the adaptor is generally referred to as a femtosecond laser, in which a pulse type laser beam irradiated through an object lens of the laser is applied to a corneal stroma in the cornea, corneal tissues are changed into gas plasma to generate micro-scale air bubbles, and a desired amount of corneal tissue is accurately incised as the result of continuous application of the laser beam.

The laser beam radiated from the femtosecond laser is transmitted through the surface of the cornea and incises a lenticule which is a portion of the corneal stroma, in accordance with a desired amount of correction, thereby correcting imperfect vision, such as hyperopia, astigmatism, myopia, presbyopia, or mixed astigmatism, which is referred to as small incision lenticule extraction (SMILE) surgery. The above SMILE surgery is regarded as the same kind of surgery even though various kinds of lasers are used and various names are used depending on the manufacturer.

Spots of the laser beam may form an optical penetration portion in the corneal stroma. Plasma bubbles are generated as the result of such penetration, so that a lenticule which is a portion of the corneal stroma is separated from the corneal stroma.

In order to incise the lenticule, a focal point of the laser beam is guided to a target in the cornea below a corneal epithelium and a Bowman's membrane and above a Descemet's membrane and a corneal endothelium. To this end, the femtosecond laser has a tool for displacing the focal point of the laser beam, the detailed description of which will be omitted herein.

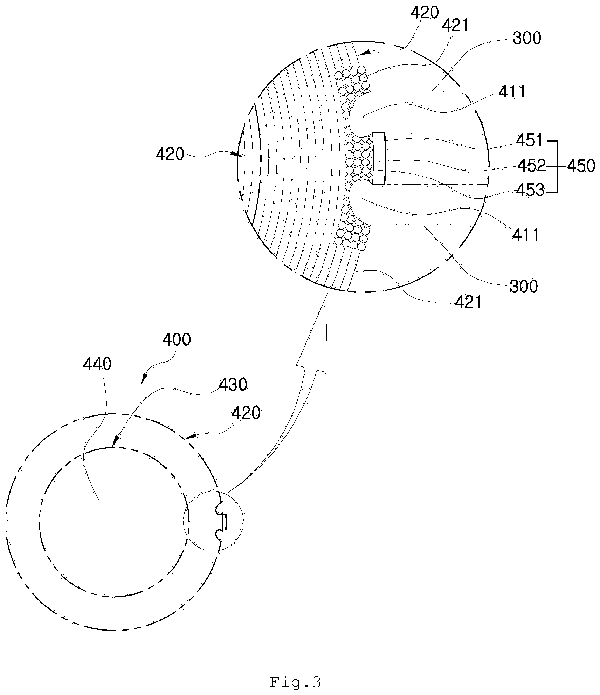

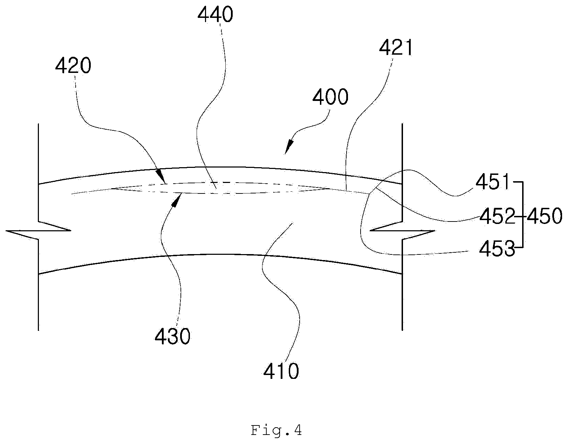

A process of displacing the focal point of the laser beam to incise the lenticule from the corneal stroma will be described with reference to Figs. 3 and 4.

A laser beam irradiated from an object lens of the laser is irradiated into a corneal stroma 410 to the center of a cornea 400 from an outside of the cornea 400 in a spiral fashion in the state in which the focal point of the laser beam is displaced, as illustrated in Fig. 3, thereby forming a flap surface 420 which is an upper separated surface that can be separated from the upper portion of the corneal stroma 410, and a lenticular surface 430 which is a lower separated surface that can be separated from the lower portion of the corneal stroma 410, with the flap surface 420 and the lenticular surface being coaxially arranged.

In this instance, the flap surface 420 is formed so as to be larger than the lenticular surface 430, and as illustrated in Fig. 4, an outer circumference of the lenticular surface 430 intersects a point inside an outer circumference of the flap surface 420 that faces the center of the flap surface 420. As a result, a three-dimensional lenticule 440 of a substantially lens shape is incised from the corneal stroma 410 by the intersected flap surface and lenticular surface.

The outer circumference of the lenticular surface 430 may be spaced apart from the outer circumference of the flap surface 420 without intersecting a point inside the outer circumference of the flap surface 420 that faces the center of the flap surface 420. In this instance, the outer circumference of the lenticular surface 430 may be connected to a point inside the outer circumference of the flap surface 420 that faces the center of the flap surface 420 through an incision surface (not illustrated) formed by the irradiation of the laser beam, the focal point of which has been displaced, thereby forming other three-dimensional lenticule from the corneal stroma 410.

If the lenticule 440 is incised from the corneal stroma 410, the lenticule 400 is withdrawn and removed from the cornea 400.

In order to withdraw the lenticule 440 out of the cornea 400, an incision portion 450 which has an incision entrance 451 and an incision tunnel 452 is formed in the cornea 400 such that a separation tool 500 for completely separating the lenticule 440 from the corneal stroma 410 or a picking tool for removing the completely separated lenticule 440 out of the cornea 400 can be inserted into or withdrawn from the cornea 400.

The incision entrance 451 is incised in a curved shape on one side of the surface of the cornea 400 to have a desired length in a circumferential direction after the laser beam irradiated from the object lens of the laser forms the lenticular surface 430 and the flap surface 420. The incision tunnel 452 facing the inside of the cornea 400 from the incision entrance 451 meets an incision line 421 positioned on the outer circumference of the flap surface 420.

The incision entrance 451 and the incision tunnel 452 which constitute the incision portion 450 may be formed in lengths of about 2 to 4 mm in the circumferential direction.

The position of the incision entrance 451 may be formed on the surface of the cornea 400 which meets on a vertical line or a line of an acute or obtuse angle relative to the outer circumference of the flap surface 420 which is formed in the corneal stroma 410.

One or two incision portions 450 may be formed on the surface of the cornea 400, and, in case of forming two incision portions 450, it may be formed symmetrically or asymmetrically relative to the center of the cornea 400.

The position or number of the incision portions 450 may be varied depending upon a surgical direction of the patient or a position of an eyeball fixing device.

The incision tunnel 452 of the incision portion 450 is a path that extends from the incision entrance 451 to the lenticule 440 in the corneal stroma 410. The separation tool 500 may be inserted into the cornea 400 through the incision entrance 451 and the incision tunnel 452 to completely separate the lenticule 440 from the corneal stroma 410. The separated lenticule 440 may be removed out of the cornea 400 through the incision tunnel 452 and the incision entrance 451 using the picking tool.

The incision tunnel 452 is formed to a predetermined depth in the corneal stroma 410 in the process of forming the incision tunnel 452 in the surface of the cornea 400 via the application of a laser beam. The laser beam is applied into the corneal stroma 410, in which the lenticule 440 is located. A front end 453 of the incision tunnel 452 is connected to the incision line of the flap surface 420.

Alternatively, the incision tunnel 452 may be formed by the separation tool 500 that is inserted into the corneal stroma 410, in which the lenticule 440 is located, through the incision entrance 451, after only the incision entrance 451 is formed by the laser beam. A front end 453 of the incision tunnel 452 may be connected to the incision line of the flap surface 420.

The incision tunnel 452 may be formed to the same length as the circumferential length of the incision entrance 451, or to a length different from the circumferential length of the incision entrance 451.

When the front end 453 of the incision tunnel 452 is connected to the incision line 421 of the flap surface 420, the front end 453 of the incision tunnel 452 may be connected to the incision line positioned on the outer circumference of the flap surface 420. Alternatively, the front end 453 of the incision tunnel 452 may be connected to other incision line positioned on the flap surface that is located at a point closer to the center of the flap surface 420 than to the outer circumference of the flap surface.

The incision tunnel 452 may be variously deformed depending on the position of the front end 453 of the incision tunnel 452 and the position of the incision entrance 451 formed on the surface of the cornea 400.

That is, the front end 453 of the incision tunnel 452 may be connected to the incision line positioned on the outer circumference of the flap surface 420, and the incision entrance 451 may be formed in the surface of the cornea 400 which meets on a vertical line at the incision line 421 positioned on the outer circumference of the flap surface 420, thereby forming the incision portion 450.

In addition, the front end 453 of the incision tunnel 452 may be connected to the incision line 421 positioned on the outer circumference of the flap surface 420, and the incision entrance 451 may be formed in the surface of the cornea 400 which meets on a line of an acute or obtuse angle relative to the incision line 421 positioned on the outer circumference of the flap surface 420, thereby forming the incision portion 450.

Furthermore, the front end 453 of the incision tunnel 452 may be connected to other incision line positioned on any one inner portion of the outer circumference of the flap surface 420 that faces the center of the flap surface 420, and the incision entrance 451 may be formed in the surface of the cornea 400 which meets on a vertical line or a line of an acute or obtuse angle relative to other incision line, thereby forming the incision portion 450.

Meanwhile, the spot of the laser beam is continuously applied into the corneal stroma 410 in a dense spiral fashion to form the lenticular surface 430 in the inner lower portion of the corneal stroma 410, and then the flat surface 420 is formed in the inner upper portion of the cornea stoma 410, so that the lenticule 410 is incised by the lenticular surface 430 and the flap surface 420. However, the lenticule 440 is not completely separated from the corneal stroma 410 due to the tissue bridges which is a region between stops of the laser beam.

Accordingly, after the separation tool 500 of a predetermined length, such as a needle, is inserted through the incision entrance 451 and the incision tunnel 452, the separation tool 500 entirely passes through the bottom surface of the lenticule 440 which is the lenticular surface 430 and the top surface of the lenticule 440 which is the flap surface 420 to cut the tissue bridges, so that the lenticule 440 is completely separated from the corneal stroma 410. After that, the separation tool 500 is withdrawn, and the picking tool, such as tweezers, is inserted through the incision entrance 451 and the incision tunnel 452 to remove the lenticule 440 which has been completely separated from the corneal stroma 410, to the outside through the incision tunnel 452 and the incision entrance 451.

When the separation tool 500 which has been inserted through the incision entrance 451 and the incision tunnel 452 cuts the tissue bridges while entirely moving along the bottom surface and the top surface of the lenticule 440, external force generated by the motion of the separation tool 500 is applied to both inner sides of the incision entrance 451 and the incision tunnel 452 in the circumferential direction thereof.

When the external force is applied to both inner sides of the incision entrance 451 and the incision tunnel 452 in the circumferential direction thereof as the result of the motion of the separation tool 500, the front end 453 of the incision tunnel 452 is positioned on the incision line of the outer circumference of the flap surface 420 which is formed by the laser beam spots applied in the spiral fashion. Consequently, the incision tunnel 452 cannot structurally withstand the external force applied to both inner sides of the incision tunnel 452 in the circumferential direction thereof. As a result, the separation tool 500 easily moves along the incision line 421, so that both inner sides of the incision tunnel 452 are easily torn.

In the case where the incision entrance 451 and the incision tunnel 452 are torn, the risk of corneal infection is increased, the medical treatment time is increased, and tissues are not stabilized, thereby extending a recovery time.

The present invention is characterized in that some regions of both sides of the front end 453 of the incision tunnel 452 which meets the incision line 421 positioned on the outer circumference of the flap surface 420 formed by the laser beam are not incised by the laser beam, in order to prevent both inner sides of the incision entrance 451 and the incision tunnel 452 in the circumferential direction thereof from being easily torn by the separation tool 500.

Specifically, as illustrated in Fig. 3, when the incision line 421 positioned on the outer circumference of the flap surface 420 is formed, a pair of laser beam non-irradiated regions 411 having a width corresponding to the circumferential length of the front end of the incision tunnel 452 connected to the incision line 421 are formed on the incision line 421 positioned on the outer circumference of the flap surface 420.

According to an embodiment to form the laser beam non-irradiated region 411 on the incision line 421 positioned on the outer circumference of the flap surface 420, as illustrated in Figs. 1a and 1b and Figs. 2a and 2b, a pair of spaced shields 300 for blocking penetration of the laser beam may be formed on a non-refraction lens 140 or a refraction lens 210 of the adaptor.

The interval between the spaced shields 300 corresponds to the circumferential length of the incision portion 450. If the pair of spaced laser beam non-irradiated regions 411 is formed on the incision line positioned on the outer circumference of the flap surface 420 by the shields 300, the front end 453 of the incision tunnel 452 of the incision portion 450 is connected to the incision line 421 positioned between the laser beam non-irradiated regions 411. Therefore, even though the external force generated by the motion of the separation tool 500 is applied to both inner sides of the incision tunnel 452 in the circumferential direction thereof, a portion which is not incised by the laser beam non-irradiated region 411 resists the external force of the separation tool 500, thereby preventing the incision entrance 451 and the incision tunnel 452 from being torn due to the motion of the separation tool 500.

Since the laser beam non-irradiated region 411 is formed by the shield 300, the laser beam non-irradiated region 411 has a shape corresponding to that of the shield 300, and the shield 300 may be provided on the top or bottom surface of the non-refraction lens 140 or refraction lens 210.

If the shield 300 is provided on the refraction lens 210, it is preferable to set the position of the shield 300 in consideration of the refractive index of the refraction lens 210 through which the laser beam goes.

The size of the lenticule 440 and the flap surface 420 may be varied depending upon a range of correction of refractive abnormalities, and the position of the shield 300 may be changed according to the varied size.

It is possible to prepare non-refraction lenses 140 or refraction lenses 210 of various kinds, of which the position of the shield 300 is different, in advance, and select and use any one of the non-refraction lens 140 and the refraction lens 210 according to the range of correction of refractive abnormalities.

The shield 300 may be configured in various shapes to block the laser beam. For example, the shield may be formed as a coating layer or a printed layer formed on the non-refraction lens 140 or refraction lens 210 by a material containing components through which the laser beam does not penetrate. The shield may be formed by corroding or eliminating a region corresponding to the laser beam non-irradiated region 411 on or from the non-refraction lens 140 or refraction lens 210. The shield may be formed by etching a region corresponding to the laser beam non-irradiated region 411 on the non-refraction lens 140 or refraction lens 210 by high-output laser beam. Alternatively, the shield may be formed by attaching an adhesive substance, such as a tape, having a shape corresponding to the laser beam non-irradiated region 411 to the non-refraction lens 140 or refraction lens 210.

The pair of shields 300 may be formed in the shape of any one of a circle, an oval, a polygon, or a straight or curved shape. The interval between the spaced shields 300 may be varied depending upon the inner circumferential length of the incision portion, and preferably has a length of 0.5 mm to 4 mm.

If the shield 300 is formed in the straight shape, the shields may be horizontally formed to the center of the cornea 400, or may have a desired inclined angle while being symmetrical to the center of the cornea.

The shields 300 may be formed in such a way that one end facing the center is simply rounded, but an inner portion of the end may protrude from a line corresponding to the incision line 421, as illustrated in Figs. 1b and 2b.

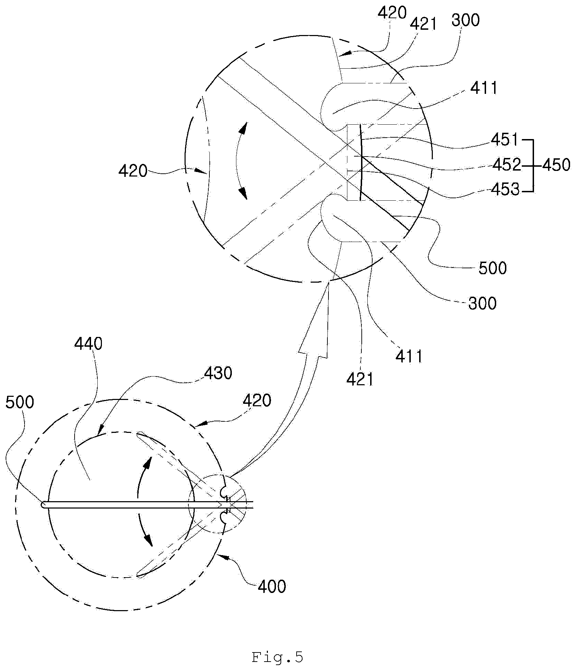

When the separation tool 500 is inserted in the incision portion 450 and then is rotated, as illustrated in Fig. 5, the separation tool 500 does not come into contact with the incision line 421, but comes into contact with the protruding portion in the non-irradiated region 411, thereby further effectively preventing the incision portion 450 from being torn.

The laser beam non-irradiated region 411 which is formed on the incision line 421 positioned on the outer circumference of the flap surface 420 and is positioned on both sides of the front end 453 of the incision tunnel 452 met the incision line 421 is formed by the shield 300 provided on the adaptor, but may be formed under control of a program related to irradiation and non-irradiation of the laser beam of the laser, without using the shield 300.

Specifically, when the incision line 421 is formed on the outer circumference of the flap surface 420 by irradiating the laser beam into the corneal stroma 410, the region corresponding to the laser beam non-irradiated region 411 may be not irradiated by the laser beam under the control of the program.

If the front end 453 of the incision tunnel 452 is positioned in the pair of laser beam non-irradiated regions 411 formed on the incision line 421 positioned on the outer circumference of the flap surface 420, the laser beam non-irradiated region 411 which is not incised serves as reinforcement for the external force applied to both inner sides of the incision tunnel 452, thereby preventing the incision entrance 451 and the incision tunnel 452 from being torn.

Therefore, in case where the laser beam non-irradiated region 411 is formed, it is possible to prevent the incision portion 450 from being damaged during the operation, even if the length of the incision entrance 451 and the incision tunnel 452 is minimized to about 0.5 mm to less than 2 mm.

Hereinafter, the major processes of the SMILE surgery using the adaptor with the shield 300 will be described briefly.

After the upper portion of the adaptor with the shield 300 provided on the bottom surface of the non-refraction lens 140 or refraction lens 210 is fixed on the object lens of the laser by partial vacuum, a bed on which a patient put under local anesthesia is placed, or the laser is moved to align the lower portion of the adaptor with the center of the cornea 400 of the patient, and then the adaptor is fixed by the partial vacuum.

If necessary, triple centration is performed in consideration of an error between a pupil axis and a visual axis. The triple centration is a technique of performing a surgical operation while being aligned with the center of the cornea 400, which may improve the accuracy of the surgical operation and may prevent blurring, photophobia, double vision, and amblyopia.

The triple centration may include a step of setting centration using a marking pen for surgical operation in consideration of the error between the pupil center and the visual axis, a step of aligning the center of the femtosecond laser with the marked area, and a step of applying the laser beam of the laser to the center of the marked area.

After the triple centration is completed, the laser beam from the laser which is programmed in accordance with a desired amount of correction is applied into the corneal stroma 410 of the cornea 400. In this instance, the laser beam transmits the non-refraction lens 140 or refraction lens 210 of the adaptor, and then a plurality of spots are continuously formed while a spiral line is drawn from the outside of the cornea 400 to the center of the cornea 400, thereby first forming the lenticular surface 430 which will be separated from the corneal stroma 410, below the corneal stroma 410.

Subsequently, the focal point of the laser beam is displaced, with the result that the focal point of the laser beam is formed a point upwardly spaced apart from the lenticular surface 430 in the corneal stroma 410, and a plurality of spots are continuously formed while drawing a spiral line from the center of the cornea 400 to the outside of the cornea 400, thereby forming the flap surface 420 which will be separated from the corneal stroma 410, above the corneal stroma 410.

In this instance, the flap surface 420 is formed so as to be larger than the lenticular surface 430, and the outer circumference of the lenticular surface 430 intersects the outer circumference of the flap surface 420 inside the outer circumference of the flap surface 420 facing the center, thereby forming the lens-shaped three-dimensional lenticule 440 having the upper flap surface 420 and the lower lenticular surface 430 which is incised from the corneal stroma 410.

When the incision line 421 positioned on the outer circumference of the flap surface 420 in the process of forming the flap surface 420 by irradiation of the laser beam passing the non-refraction lens 140 or refraction lens 210 is formed, a portion of the incision line 421 becomes a pair of laser beam non-irradiated regions 411 which is not incised, by the shield 300 formed on the non-refraction lens 140 or refraction lens 210.

After the incised lenticule 440 is completely separated from the corneal stroma 410, the incision entrance 451 having the desired length which is curved in the circumferential direction, and the incision tunnel 452 extending from the incision entrance 451 and having the front end 453 connected to the incision line 421 positioned between the laser beam non-irradiated regions 411, among the incision lines positioned on the outer circumference of the flap surface 420, are formed on the surface of the cornea 400 which is opposite to the outer circumference of the flap surface 420 to withdraw the lenticule 400 out of the cornea 400.

If the laser beam non-irradiated regions 411 are formed on the incision line 421 positioned on the outer circumference of the flap surface 420, and the front end 453 of the incision tunnel 452 is connected between the laser beam non-irradiated regions 411, even though the interval between the laser beam non-irradiated regions 411, the incision tunnel 452 positioned between the laser beam non-irradiated regions 411, and the incision entrance 451 connected to the incision tunnel 452 are formed to a length of about 2 mm, it is possible to prevent the incision entrance 451 and the incision tunnel 452 from being torn due to rotation and movement of the separation tool 500 on the lenticular surface 430 and the flap surface 420 through the incision entrance 451 and the incision tunnel 452.

The interval between the laser beam non-irradiated regions 411 and the interval between the incision entrance 451 and the incision tunnel 452 are not necessarily defined by any one, but may have a minimum length sufficient to allow the separation tool 500 to be inserted therethrough. For example, the interval may be formed to a length of about 0.5 mm to 2 mm.

The laser beam non-irradiated region 411 is formed on the incision line 421 positioned on the outer circumference of the flap surface 420. After the front end 453 of the incision tunnel 452 is connected to the incision line 421 between the laser beam non-irradiated regions 411, the separation tool 500 is inserted through the incision entrance 451 and the incision tunnel 452, and completely separates the lenticular surface 430 and the flap surface 420 which have not been completely separated from the corneal stroma 410 due to tissue bridges, from the corneal stroma 410.

Specifically, as illustrated in Fig. 5, the separation tool 500 is inserted into the incision line 421 positioned between the laser beam non-irradiated regions 411 through the incision entrance 451 and the incision tunnel 452, and is then rotated along the lenticular surface 430 in bilateral directions to completely separate the lenticular surface 430 from the corneal stroma 410.

In this instance, the surgical operation may be performed without using an eyeball fixing device in order to minimize bleeding, congestion, and pain.

After the lenticular surface 430 is completely separated from the corneal stroma 410, the separation tool 500 is completely withdrawn from the incision entrance 451, and then is inserted into the incision line 421 positioned between the laser beam non-irradiated regions 411 through the incision entrance 451 and the incision tunnel 452 to completely separate the flap surface 420 from the corneal stroma 410 by rotating the separation tool in bilateral directions along the flap surface 420.

Alternatively, after the lenticular surface 430 is separated, the separation tool 500 may be minutely lifted in the incision tunnel 452 without completely withdrawing the separation tool 500 from the incision entrance 451 to complete the separation of the flap surface 420 from the corneal stroma 410, which can shorten the operation time. And, the movement of the separation tool 500 is minimized to prevent the stability of the operation and damage of the incision portion.

In the process of rotating and moving the separation tool 500 which has been inserted into the incision entrance 451 and the incision tunnel 452, in bilateral directions to completely separate the lenticular surface 430 and the flap surface 420 from the corneal stroma 410, even though the external force is applied to both inner sides of the incision tunnel 452 by the motion of the separation tool 500 in the incision tunnel 452, the separation tool 500 comes into contact with the portions protruding inwardly from the laser beam non-irradiated region 411 which is not incised on the incision line 421, thereby preventing both inner circumferential sides of the incision tunnel 452 from being torn. In addition, it is also possible to prevent both circumferential sides of the incision entrance 451 from being torn when the incision tunnel 452 is torn.

Since the strength of both inner circumferential sides of the incision tunnel 452 is increased by the laser beam non-irradiated region 411 which is not incised, therefore, it is possible to prevent the incision entrance 451 and the incision tunnel 452 from being torn due to the motion of the separation tool 500 which is inserted through the incision portion 450 even though the circumferential incision length of the incision entrance 451 and the incision tunnel 452 is minimized to 0.5 mm to 2 mm. The minimum incision of the incision portion 450 is advantageous in reducing the risk of corneal infection, reducing the medical treatment time, and stabilizing tissues.

After the lenticule 440 is completely separated from the corneal stroma 410 using the separation tool 500, a picking tool is inserted into the corneal stroma 410, in which the lenticule 440 is located, through the incision entrance 451 and the incision tunnel 452 to pick the separated lenticule 440 and to remove the picked lenticule 440 out of the corneal stroma 410 through the incision entrance 451. After that, eye drops are supplied through the incision portion 450, and then suspended matters are removed using the separation tool 500.

As described above, the present invention provides the adaptor of the laser for use in vision correction surgery that forms the laser beam non-irradiated region, wherein when the lenticule 440 having the lenticular surface 430 and the flap surface 420 is incised in the corneal stroma 410 by applying the laser beam while three-dimensionally displacing the focal point of the laser beam, and the incised lenticule 440 is removed out of the cornea through the incision portion 450 having the incision entrance 451 and the incision tunnel 452, the laser beam non-irradiated region 411 is formed on the incision line 421 positioned on the outer circumference of the flap surface 420 connected to the front end 453 of the incision tunnel 452 to include the front end 453 of the incision tunnel 452, thereby preventing the incision entrance 451 and the incision tunnel 452 from being torn due to the rotating separation tool 500 which is inserted into the incision portion 450. Consequently, the circumferential incision length of the incision entrance 451 and the incision tunnel 452 is minimized to 0.5 mm to 2 mm, thereby reducing the risk of corneal infection, reducing the medical treatment time, which reduces the inconvenience to a patient, and rapidly regenerating and stabilizing tissues, which improves the surgical operation and the patient's satisfaction.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

### [Industrial Applicability]

Since the adaptor of the laser for use in vision correction surgery according to the present invention includes the shield for blocking the laser beam, the laser beam non-irradiated regions are formed on both sides of the incision portion in the circumferential direction which is formed on the cornea to remove the three-dimensionally incised lenticule out of the cornea, thereby preventing the incision portion from being easily torn due to the rotating separation tool which is inserted into the incision portion, and thus reducing the risk of corneal infection which is caused by breakage of the incision portion, reducing the medical treatment time, and effectively stabilizing tissues. Consequently, the present invention is very useful from the aspect of industrial applicability.

## Claims

1. An adaptor of a laser for use in vision correction surgery that forms a laser beam non-irradiated region, of which an upper portion is coaxially aligned with an object lens of the laser, and a lower portion is coaxially aligned with a cornea of a patient, in order to set a position of the cornea of the patient with respect to the object lens of the laser,
wherein a pair of spaced shields for blocking downward penetration of the laser beam is provided on a non-refraction lens or refraction lens which is installed in the adaptor, the laser beam of the laser going through the non-refraction lens or refraction lens.

2. The adaptor of the laser according to claim 1, wherein a focal point of the laser beam is three-dimensionally displaced to continuously draw dense helices on an inside of the cornea, so that a flap surface and a lenticular surface which have a continuous spiral incision line are formed in a corneal stroma,
an outer circumference of the lenticular surface intersects at any point inside an outer circumference of the flap surface, and the laser beam non-irradiated region is formed by the shield when a three-dimensional lenticule is incised from the corneal stroma, and
the laser beam non-irradiated region is formed on any one of the incision line of the flap surface which is positioned between outer circumferential incision lines, among outer circumferential incision lines of the flap surface.

3. The adaptor of the laser according to claim 2, wherein an incision entrance having a desired length in a circumferential direction is formed on the surface of the cornea which is opposite to the incision line, by the laser beam irradiated from the laser, a front end of an incision tunnel formed toward the corneal stroma is connected to the incision line of the flap surface, and the front end of the incision tunnel is positioned between the laser beam non-irradiated regions positioned on the incision line of the flap surface.

4. The adaptor of the laser according to claim 3, wherein the incision entrance is formed in the surface of the cornea which is positioned on any one of a vertical line or a line of an acute or obtuse angle relative to the outer circumferential incision line of the flap surface.

5. The adaptor of the laser according to claim 1, wherein the shield is formed on a bottom surface of the non-refraction lens or refraction lens.

6. The adaptor of the laser according to claim 1, wherein the shield includes any one of a coating layer, a printed layer, a etched portion, and an adhesive substance.

7. The adaptor of the laser according to claim 1, wherein the shield includes any one of a pair of circular shields, a pair of oval shields, a pair of polygonal shields, a pair of straight shields, and a pair of curved shields.

8. The adaptor of the laser according to claim 2, wherein an interval between the spaced shields has a length of 0.5 mm to 4 mm.

9. The adaptor of the laser according to claim 7, wherein the shield includes a pair of straight shields which is horizontal to each other or has an inclined angle while being symmetrical to each other.

10. The adaptor of the laser according to claim 7, wherein the shield is formed in such a way that an inner portion of an end facing a center protrudes in an inward direction.
